# EUROPÄISCHE PATENTSCHRIFT

(11) **EP 0 755 530 B1**
(45) Veröffentlichungstag und Bekanntmachung des Hinweises auf die Patenterteilung: **07.06.2000**
(21) Anmeldenummer: 95914354.6
(22) Anmeldetag: 09.04.1995
(51) Int. Cl.: G02B 21/00, G01B 9/04

(54) **Anordnung zur Positionsbestimmung eines Operationsmikroskops**
Device for determining the position of a surgical microscope
Dispositif de détection de la position d'un microscope chirurgical

(30) Priorität: 11.04.1994 CH 108894; 11.04.1994 CH 108994; 11.04.1994 CH 109094; 11.04.1994 CH 109194; 11.04.1994 CH 109294
(43) Veröffentlichungstag der Anmeldung: 29.01.1997
(60) Teilanmeldung: 97119033.5
(73) Patentinhaber: Leica Microsystems AG, 9435 Heerbrugg (CH)
(72) Erfinder: SPINK, Roger, CH-9436 Balgach (CH); BRAUNECKER, Bernhard, CH-9445 Rebstein (CH); ZIMMER, Klaus-Peter, CH-9435 Heerbrugg (CH); MAYER, Thomas, A-6845 Hohenems (AT); ROGERS, John, Rice, CH-9435 Heerbrugg (CH)
(74) Vertreter: Stamer, Harald, Dipl.-Phys.
(86) Internationale Anmeldenummer: EP9501311
(87) Internationale Veröffentlichungsnummer: WO9527918

(56) Entgegenhaltungen:
- EP-A- 0 164 680
- EP-A- 0 456 103
- WO-A-88/07312
- WO-A-93/16631
- DE-A- 4 134 481
- DE-A- 4 202 505
- NL-A- 9 000 622
- US-A- 4 122 518
- US-A- 4 638 471
- US-A- 4 672 559
- US-A- 4 686 639
- US-A- 5 098 426
- US-A- 5 108 174
- US-A- 5 249 581
- US-A- 5 279 309

## Beschreibung

Die Erfindung betrifft eine Anordnung zur Positionsbestimmung eines Operationsmikroskopes im Raum. Die Positionsbestimmung des Mikroskopes wird dabei deshalb vorgenommen, um bei gleichzeitiger Kenntnis der Position eines Patienten im Raum, aus den Einstellungsdaten des Mikroskopes (Fokus, Zoom etc.) auf den genauen Ort des durch das Mikroskop betrachteten Operationsfeldes zu schliessen. Dies wird in der modernen Mikrochirurgie (Stereotaxie) immer wichtiger, da es so einem Operateur ermöglicht wird, den Ort, an dem er die Operation durchzuführen hat, mit grosser Sicherheit zu finden. Umgekehrt kann auf diese Art und Weise auch genau verfolgt werden, an welchem Ort die Operation durchgeführt wird. Die Bedeutung solcher Kenntnisse steigt mit der fortschreitenden Technik, unterschiedliche Diagnosebilddaten, z.B. Röntgen-, CT- oder MRI-Bilddaten in einem Mikroskopstrahlengang sichtbar zu machen, bzw. dem gesehenen Bild zu überlagern, so dass der Operateur auch entsprechende Vergleichsinformationen - ortsmässig richtig dargestellt - heranziehen kann.

Die Positionsbestimmung des Mikroskopes (Im Sinne der Erfindung kann damit auch ein Endoskop gemeint sein) erfolgt nach dem Stand der Technik mit verschiedenen Techniken. Einerseits gibt es Mikroskopträgergestelle, die in ihren Gelenken über Messelemente verfügen - ähnlich einem Roboterarm aus der Maschinenindustrie - die Lageveränderungen des Mikroskopes ständig mitverfolgen und über einen Mikroprozessor die jeweilige Position im Raum ermitteln.

Aus der DE 42 02 505 A1 ist ein Führungssystem zur kontaktlosen Führung und Positionierung eines Operationsmikroskops durch die Kopfbewegungen des mit dem Operationsmikroskop arbeitenden Chirurgen bekannt. Dazu wird eine optische Sende- und Empfangseinheit verwendet, die wechselweise gegenüber am Kopf des Chirurgen und am Operationsmikroskop angebracht sind. Darüber hinaus können auch Sende- und Empfangseinheit zusammen angeordnet sein, wobei ihnen Reflektorelemente gegenüberliegen. Die von der Sendeeinheit kommenden Strahlen werden von den Reflektorelementen in die Empfangseinheit reflektiert. Die Reflektorelemente sind dabei für eine herkömmliche Reflexion der Strahlen ausgelegt. Eine zusätzliche Beeinflussung der Strahlen an den Reflektorelementen in irgendeiner Art ist dabei nicht vorgesehen. Mit Hilfe der von den Detektoren registierten Auslenkungen der auftreffenden Strahlenbündel von definierten Soll-Auftreffpunkten wird die veränderte Kopfposition des Chirurgen erfaßt und das Operationsmikroskop entsprechend nachgeführt.

Andererseits gibt es auch Mikroskope und Endoskopspitzen, die mit Hilfe von Ultraschallsendern und -sensoren, die am Mikroskop angeordnet sind, die jeweilige Position ermitteln. Bei Endoskopen wird fallweise auch unter einem permanenten Röntgenstrahl gearbeitet, um die Lage wenigstens optisch sichtbar bestimmen zu können.

Bei einer neuen Entwicklung der Anmelderin wird auf die Ultraschalltechnik verzichtet und ein Infrarotpositioniersystem angewendet, bei dem am Mikroskop drei Infrarotsender mit kodierten Sendesignalen angeordnet sind, deren Signale von im Operationsraum angeordneten Infrarotempfängern detektiert werden. Diese Technik erlaubt eine exaktere Positionsbestimmung als mittels Ultraschall, da vor allem auch Störeinflüsse ausgeschlossen werden können und das gleichzeitige Arbeiten mit Ultraschallgeräten - wie es bei Operationen im Bereich des Gehirnes vorkommen kann - nicht kontraindiziert ist.

Bei allen bisher bekannten Positionsbestimmungsverfahren sind trotz grundsätzlicher Funktionstüchtigkeit der Verfahren zwei Probleme zu beachten: Der Patient sollte durch die Positionsbestimmung nicht beeinflusst werden und allfällige Messergebnisse im Bereich der Operationsstelle sollten durch das Positionsbestimmungsverfahren nicht beeinflusst werden.

Diese Probleme sind nicht zufriedenstellend gelöst. Der speziellen Ausgestaltung liegt daher die Aufgabe zugrunde, ein Operationsmikroskop mit einem Positionsermittlungssystem zu schaffen, das weder den Patienten noch allfällige Messergebnisse aus dem Bereich der Operationsstelle (z.B. Gehirnstrommessungen) beeinflusst.

Gelöst wird diese Aufgabe durch das Verwenden von schall- und stromunabhängigen Reflektoren mit spezifischen Reflexionseigenschaften am Operationsmikroskop, wodurch die Signale der Sender reflektorspezifisch von den Empfängern detektiert werden.

Bevorzugt ist dabei der Aufbau mit Infrarotsendern, die vom Operationsmikroskop entfernt angeordnet, mittels Strom erregt werden. Die von ihnen abgestrahlten Lichtimpulse können auch mittels Glasfasern bis zum Mikroskop geleitet werden, um dort die Glasfaserenden zu verlassen und - wie bei der Anordnung mit IR-Leuchtdioden am Mikroskop - von den Sensoren im Raum erfasst zu werden und eine entsprechende Positionsbestimmung zu ermöglichen.

Besondere Ausführungsformen dieser Variante sind in den abhängigen Patentansprüchen beschrieben bzw. unter Schutz gestellt. Sie erleichtern vor allem das Reduzieren der Baugrösse von Operationsmikroskopen.

Diese Anordnung führt somit zu kompakteren Operationsmikroskopen, deren Position im Raum einfach bestimmbar ist, ohne störende Ultraschallsignale, Stromflüsse oder elektromagnetische Felder. Dem Operateur ist somit ein patientenfreundlicheres und ortspräziseres Arbeiten möglich.

Im Rahmen der Erfindung liegen darüber hinaus verschiedene Ausbildungsarten und Varianten dazu, die sich aus er Kombination der hier erwähnten Merkmale mit Merkmalen der stamm-Anmeldung WO-A-95 27 918 ergeben, die andere Aspekte eines erfinderischen neuen Mikroskopes unter Schutz stellen, das bevorzugt eben auch mit der vorliegend beschriebenen Anordnung ausgerüstet ist. Weitere Aspekte ergeben sich noch aus den zeitrangig gleichen Patentanmeldungen WO-A-95 27 226 und WO-A-95 27 917 der Anmelderin.

Weitere Details und Ausführungen der Erfindung ergeben sich aus der Zeichnung. Die dort dargestellten Figuren zeigen:
- Fig.1: einen Aufbau zur Erfassung der Mikroskopposition im Raum;
- Fig.2: eine Detailvariante zu Fig.1 und
- Fig.3: eine weitere Variante dazu.

Die Figuren werden zusammenhängend beschrieben. Gleiche Bezugszeichen bedeuten gleiche Bauteile. Gleiche Bezugszeichen mit unterschiedlichen Indizes bedeuten ähnliche bzw. funktionsähnliche Bauteile. Die Erfindung ist auf die dargestellten Ausführungsbeispiele nicht eingeschränkt. Vor allem in Kombination mit den Lehren der oben angeführten PCT-Patentanmeldungen lassen sich noch beliebige Varianten darstellen. Sie alle fallen unter den Offenbarungsinhalt dieser Anmeldung.

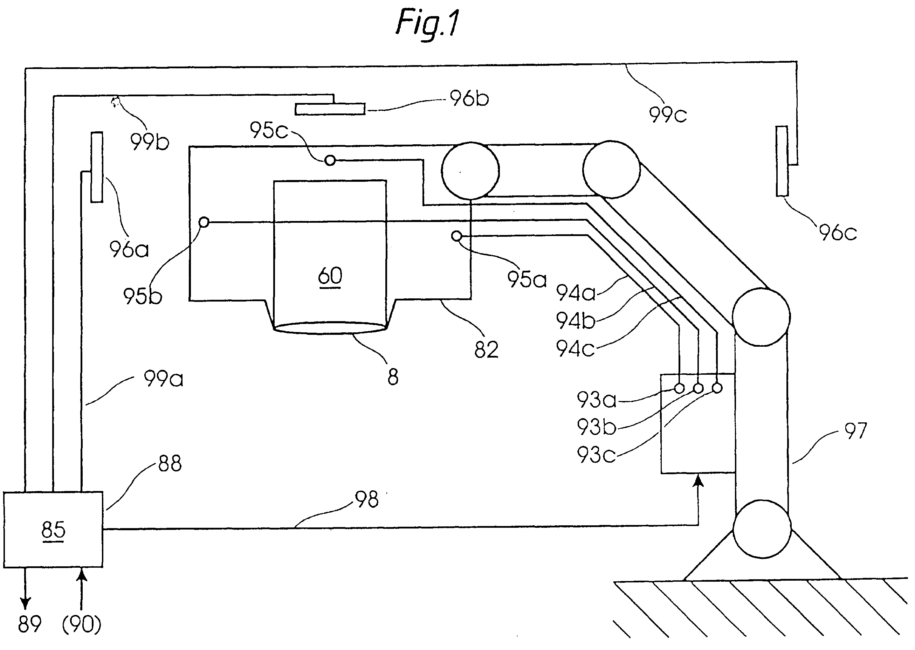

Das Prinzip der Erfindung wird in Fig.1 verdeutlicht: Mehrere peripher angeordnete Leuchtdioden 93 senden kodierte Lichtimpulse, die über Glasfaserleitungen 94 an das Mikroskop 82 geleitet werden. Aus den Enden 95 der Glasfasern 94 treten die Lichtimpulse aus und können so von im Raum angeordneten IR-Sensoren 96 erfasst werden. Die eingehenden Impulse werden dann über Zuleitungen 99 geführt und von einer Auswerteeinheit 85 ausgewertet und die Position des Mikroskopes 82 derart bestimmt. Die Auswerteeinheit 85 ist mit einer Datenaufbereitungseinheit 89 oder ggf. direkt mit einem Computer 90 verbunden. Die Auswerteeinheit 85 kann über eine Rückkopplungsverbindung 98 die Leuchtdioden 93 ansteuern. Das Mikroskop 82 enthält ein Objektiv 8 und einen Strahlengang 60. Es wird über einen Mikroskopständer 97 gehalten.

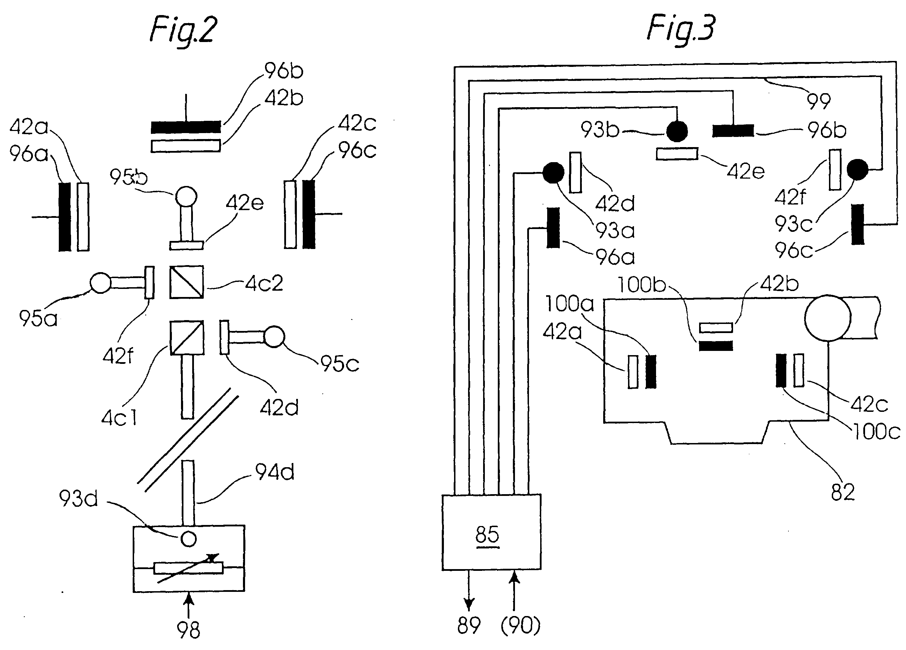

In der Variante nach Fig.2 wird nur das Licht einer einzigen Leuchtdiode 93d benutzt, die allerdings abwechselnd in verschiedenen Lichtfarben Lichtimpulse abstrahlt. Dies geschieht beispielsweise durch Variation der Speisespannung der Leuchtdiode 93d. Am mikroskopseitigen Ende der Glasfaser 94d sind zwei Strahlenteiler 4c1 und 4c2 angeordnet, die das ankommende Lichtbündel auf drei Messbündel aufteilen. diese werden durch geeignete schmalbandige Farbfilter 42 jeweils nur zu den zugehörigen Glasfaserenden 95 durchgelassen, wodurch eine räumliche Auftrennung der abgehenden Lichtimpulse möglich ist.

Entsprechende Farbfilter 42 sind den IR-Rezeptoren 96 vorgeschaltet, so dass diese auch jeweils nur auf die für sie bestimmten Lichtimpulse ansprechen.

Fig.3 zeigt eine Variante, bei der auch auf die Glasfaserleitungen verzichtet wird, indem sowohl Lichtimpulssender 93 als auch IR-Rezeptoren 96 im Raum angeordnet sind und am Mikroskop 82 lediglich Reflektoren 100 mit je einer ganz charakteristischen Reflexionseigenschaft für eine spezielle Lichtfarbe angebracht sind, denen Farbfilter 42 vorgeschaltet sind, so dass nur die entsprechende Lichtfarbe auf sie auftreffen und sie wieder verlassen kann. In einem etwas aufwendigeren Auswerteverfahren kann derart die Positionsbestimmungseinheit 85 die Position des Mikroskopes bestimmen.

Selbstverständlich können auch die Zuleitungen 99 zu den IR-Rezeptoren als Glasfasern gebildet sein, so der Raum insgesamt mit weniger elektromagnetischen Feldern belastet werden soll.

Eine bevorzugte Ausbildung betrifft somit eine Anordnung zur Positionsbestimmung eines Operationsmikroskopes 82 mit Hilfe von frequenten Lichtimpulsen, die vom Mikroskop 82 abgehen und von Lichtrezeptoren 96 empfangen werden, wobei zwischen Leuchtdioden 93 und dem Absendeort 95 der Lichtimpulse Glasfasern angeordnet sind, so dass der Bereich des Mikroskopes frei von störenden elektrischen und elektromagnetischen Wellen ist.

## Patentansprüche

1. Anordnung zur Positionsbestimmung eines Operationsmikroskopes (82) mit Signalsendern und Signalempfängern und mit an verschiedenen Orten im Raum montierten Leuchtdioden (93) zur Abgabe von kodierten Lichtsignalen und Lichtrezeptoren (96) zum Empfang der Lichtsignale, **dadurch gekennzeichnet, dass** am Operationsmikroskop (82) Reflektoren (100) angeordnet sind, die auf spezielle schmalbandige Lichtimpulse spezifische Reflexionseigenschaften aufweisen, so dass das von ihnen reflektierte Licht entsprechend detektiert werden kann.

2. Anordnung nach Anspruch 1, **dadurch gekennzeichnet, dass** den Reflektoren (100) und/oder den Lichtrezeptoren (96) und oder den Leuchtdioden (93) schmalbandige Farbfilter (42) vorgeschaltet sind.

3. Anordnung nach einem der Ansprüche 1 oder 2, **dadurch gekennzeichnet, dass** die Leuchtdioden (93) und die Lichtrezeptoren (96) im Bereich des Infrarotlichtes arbeiten.

4. Anordnung nach einem der Ansprüche 1 bis 3, **dadurch gekennzeichnet, dass** den Lichtrezeptoren (96) und/oder den Leuchtdioden (93) Lichtwellenleiter vorgeschaltet sind.

5. Anordnung nach Anspruch 4, **dadurch gekennzeichnet, dass** die Enden (95) der Lichtwellenleiter selbst linsen- oder streuelementförmig ausgebildet sind oder mit Linsen- oder Streuelementen einstückig ausgebildet sind.

## Claims

1. Arrangement for positional determination of a surgical microscope (82) with signal transmitters and signal receivers and with luminescent diodes (93), which are mounted at different locations in space, for the issue of coded light signals and light receivers (96) for receiving the light signals, characterised in that reflectors (100) are arranged at the surgical microscope (82) and have reflection properties specific to special harrow-band light pulses so that the light reflected by them can be correspondingly detected.

2. Arrangement according to claim 1, characterised in that narrow-band colour filters (42) are connected in front of the reflectors (100) and/or the light receivers (96) and/or the luminescent diodes (93).

3. Arrangement according to one of claims 1 and 2, characterised l that the luminescent diodes (93) and the light receivers (96) operate in the range of infrared light.

4. Arrangement according to one of claims 1 to 3, characterised in that optical conductors are connected upstream of the light receivers (96) and/or the luminescent diodes (93).

5. Arrangement according to claim 4, characterised in that the ends (95) of the optical conductor are constructed to be lens-shaped or dispersion-element-shaped themselves or are integrally constructed with lenses or dispersion elements.

## Revendications

1. Agencement pour la détermination de la position d'un microscope chirurgical (82) avec des émetteurs de signaux et des récepteurs de signaux et avec des photodiodes (93) montées en divers emplacements dans l'espace pour l'émission de signaux lumineux codés et des récepteurs de lumière (96) pour la réception des signaux lumineux, caractérisé en ce que des réflecteurs (100) sont agencés sur le microscope chirurgical (82), qui présentent des propriétés spécifiques de réflexion des impulsions lumineuses sur bande étroite particulières de façon que la lumière qu'ils réfléchissent puisse être détectée de manière correspondante.

2. Dispositif selon la revendication 1, caractérisé en ce que les réflecteurs (100) et/ou les récepteurs de lumière (96) et/ou les photodiodes (93) sont précédés de filtres de couleur à bande étroite (42).

3. Dispositif selon l'une des revendications 1 ou 2, caractérisé en ce que les photodiodes (93) et les récepteurs de lumière (96) fonctionnent dans le domaine de la lumière infrarouge.

4. Dispositif selon l'une des revendications 1 à 3 caractérisé en ce que les récepteurs de lumière (96) et/ou les photodiodes (93) sont précédés de conducteurs d'ondes lumineuses.

5. Dispositif selon la revendication 4 caractérisé en ce que les extrémités (95) des conducteurs d'ondes lumineuses sont elles-mêmes configurées en forme de lentille ou d'élément dispersant et/ou sont configurées en une pièce avec des lentilles ou éléments dispersants.
